# EUROPEAN PATENT SPECIFICATION

(11) **EP 0 429 975 B1**
(45) Date of publication and mention of the grant of the patent: **16.02.1994**
(21) Application number: 90121880.0
(22) Date of filing: 15.11.1990
(51) Int. Cl.: C07C 43/29, C07C 41/22

(54) **Process for the preparation of 4,4'-dibromodiphenylether**
Verfahren zur Darstellung von 4,4'-Dibrom-Diphenylether
Procédé de préparation de 4,4'-dibromo-diphényl-éther

(30) Priority: 27.11.1989 IL 92465
(43) Date of publication of application: 05.06.1991
(73) Proprietor: Bromine Compounds Ltd., Beer-Sheva 84101 (IL)
(72) Inventor: Oren, Jacob, Qiryat Bialik (IL); Hermolin, Joshua, Ramat-Hasharon (IL)
(74) Representative: Rambelli, Paolo

(56) References cited:
- DE-A- 1 930 594
- DE-B- 161 547
- US-A- 4 835 322

## Description

The present invention relates to a process for producing 4,4'-dibromodiphenylether. More particularly, the invention relates to the bromination of diphenylether in the presence of oxidizing agents in mixed solvents comprising water and water - immiscible solvents.

### The Prior Art

4,4'-dibromodiphenylether, hereafter referred to as DBDPE for the sake of brevity, has been produced in the art by brominating diphenylether in a variety of solvents, including carbon disulfide, carbon tetrachloride or carbon disulfide in the presence of iodine and bromination with bromine in glacial acetic acid in the presence of CuCO₃. Furthermore, DBDPE has been prepared in a number of other ways which do not involve bromination of diphenylether.

German Offen. 1,930,594 describes the preparation of DBDPE by the bromination of diphenylether in liquid sulphur dioxide at temperatures below 0°C. U.S. Patent 4,835,322 describes the preparation of DBDPE by reacting an excess of bromine with diphenylether without a catalyst or a solvent, followed by digestion with methanol to dissolve the impurities. This process is said to provide high purities of the final product, >99%.

The processes of the art present several disadvantages, particularly because they require excess bromine which is wasted, and because laborious work-up procedures are required, in order to obtain a final product with high purity. It is an object of the present invention to provide a process which overcomes the disadvantages of the prior art, and which can be used to prepare DBDPE in high purity and high yield.

### SUMMARY OF THE INVENTION

It is another object of the invention to provide a process by means of which diphenylether can be reacted either with bromine or hydrobromic acid, to yield the final product in a relatively simple and inexpensive process. The process according to the invention comprises reacting diphenylether with bromine and/or hydrobromic acid in the presence of an oxidizing agent, the reaction being carried out in a solvent comprising water and a water-immiscible organic solvent. The process of the invention can be carried out in a wide range of temperatures. Typically, -10° - 100°C is a permissible range of temperatures. However, as will be understood by a skilled chemist, too low temperatures may result in a reduced reaction rate, while a too high temperature affects the selectivity of the bromination and leads to undesired by-products. Preferred temperatures, therefore, lie in the range 0° - 70°C, although, as said, this is not a limiting temperature range.

Preferably, the oxidizing agent is selected from H₂0₂ and NaBrO₃. While these are convenient and preferred oxidizing agents, different oxidizing agents known in the art, such as ClO-, ClO₃-, etc., can be employed. The process of the invention can be carried out using a large variety of organic solvents. The main requirements from the organic solvent are that it is substantially non-miscible with water, and that it substantially does not react with the reactants or the product of the reaction. Preferred organic solvents of the invention will be selected from the aliphatic or the aromatic hydrocarbons. As will be apparent to a skilled person, mixtures of different organic solvents can also be employed. Of the above-mentioned organic solvents, 1,2-dichloroethane and dichloromethane are particularly convenient.

According to the present invention, it is possible to employ all the available bromine. During the bromination of diphenylether, (DPE) HBr is formed according to the equation:

3 DPE + 6 Br₂ = 3 DBDPE + 6 HBr (1).

If the oxidizing agent is employed, bromine, which will otherwise be lost as HBr, can be regenerated to a free bromine form, as exemplified in eqns. (2) and (3) below.

6 HBr + 3 H₂O₂ = 3 Br₂ + 6 H₂O (2)

6 HBr + NaBrO₃ = 3 Br₂ + 3 H₂O + NaBr (3)

The use of other oxidizing agents will of course lead to very similar reactions.

As will be apparent to the skilled chemist, the mixed solvent of invention achieves some important results. First of all, water is formed in the reaction mixture or during the recycle of the HBr, if the two stages are effected separately, but this does not constitute a problem, since the use of these oxidants is preferably carried out in the presence of water. On the other hand, the presence of organic solvents in which the product dissolves allows the bromination to be performed at relatively low temperatures, at which temperatures the selectivity of the desired product, as well as the reaction rate, are optimized. Since the organic solvent and the products are water-immiscible, and since the residual Br⁻ is water soluble, all work-up operations are simplified and permit the use of the excess HBr, which has not been reacted with the oxidizing agent, in the next batch.

Selecting the organic solvent is well within the scope of the chemist versed in the art of bromination. It may be mentioned that halogenated solvents such as CH₂Cl₂, CH₂ClCH₂Cl, CH₂Br₂, CCl₂ CCl₂ and C₆H₅Cl are particularly suitable for the process of the invention, although different solvents can also be employed.

The oxidizing agent can be introduced at the end of the bromination of diphenylether, in which case, ideally, only half the amount of bromine which is normally required when oxidizing agents are not used, can be introduced. However, it can be convenient to add the oxidizing agent to the reaction mixture at the beginning or during the reaction with bromine, because liberation of bromine from hydrobromic acid is thus achieved during bromination, and considerable time is saved. It is however clear that the oxidizing agent may be used at any convenient stage to react with HBr to liberate the desired bromine, whenever HBr becomes available before or during the reaction.

As stated before, the reaction temperature is important as it influences both the reaction rate and the selectivity of the reaction. Using the oxidizing agent and a suitable solvent in combination, particularly a solvent having a melting point below the melting point of either diphenylether or DBDPE, allows the bromination to take place at low temperatures at which the reaction rate and selectivity can be optimized. Normally, a purity of 99.0% or higher is sufficient, and a product having such a purity need not be further purified. This, as will be easily understood, results in a considerable economic advantage as compared to the known art, because costly operations are dispensed with. Reference can be made to US Patent 4,835,322, in which elevated temperatures, above the melting point of the reaction mixture, must be employed.

The work-up procedure required according to the invention is very simple and inexpensive, inasmuch as two simple steps are required. 1) the separation of the aqueous phase from the organic phase, and the potential recycle to the next batch; and 2) the evaporation and the recovery of the organic solvent from the product. The recovery and the waste treatment procedures are also much simpler and reduced, as compared to the known art, which of course results in an additional saving.

Other characteristics and advantages of the process of the invention will be further illustrated in the following examples, which are not intended to constitute a limitation of the invention.

### Example 1 (comparative)

### Bromination of DPE using 1,2-dichloroethane (EDC) as the solvent

To a four-necked round-bottomed flask equipped with a stirrer, a condenser, a dropping funnel and a thermometer, containing a stirred solution of diphenylether (170 g, 1.0 mole) in 1,2-dichloroethane (EDC) (300 ml), there were added 336 g. (2.1 moles) of Br₂. To the top of the condenser there was attached a trap to absorb HBr released during the reaction. The addition of the Br₂ took place over one hour at 5°C, after which the reaction mixture was stirred at 50°C for a further one hour. The progress of the reaction and its completion were monitored by GC. Excess Br₂ and traces of HBr were neutralized with 10% ammonia (70ml). The phases were separated and the organic layer was rinsed with water (100ml). After the distillation of the solvent from the organic phase, crude 4,4'-dibromodiphenylether (320.2g) was obtained in a yield of 97% and a selectivity of 99%.
GC analyses were conducted on a Varian Model 3400. The column used was an HP-1 (100% dimethyl polysiloxane) Megabore 5m x 0.53mm at a flow of 8ml/min. The conditions were an initial temperature of 110°C for one minute then a rise to 250°C at 15 deg./min. giving a retention time for the 4,4'-dibromodiphenylether of 7.6 min.

### Example 2

### Bromination of DPE in EDC/H₂O in the presence of 30% H₂O₂

Bromine (176g, 1.1 moles) was added to a stirred mixture of diphenylether (170g, 1.0 mole) in 1,2-dichloroethane (EDC) (300ml) and water (200ml) in a four-necked round-bottomed flask equipped with a stirrer, a condenser, a dropping funnel and a thermometer. The addition of the Br₂ took place over one hour at 5°C, without any noticeable liberation of HBr from the reaction apparatus. Thereafter, 30% H₂O₂ (130g, 1.15 moles) was added gradually to the reaction mixture at 50°C for a further one hour.
The progress of the reaction and its completion were monitored by GC. Full conversion was achieved after three hours, after which the phases were separated.
After distillation of the solvent, crude 4,4'-dibromodiphenylether was obtained (318.1g) in a yield of 96% and a selectivity of 99%.

Comparison of these results and those obtained in Example 1 shows that almost identical yields and selectivities are obtained with about 50% Br₂.

### Example 3

### Bromination of DPE in EDC/H₂O in the presence of NaBrO₃

Bromine (176g, 1.1 moles) was added to a stirred mixture of diphenylether (170g, 1.0 mole) in 1,2-dichloroethane (EDC) (300ml), water (200ml) and NaBrO₃ (55g, 0.36 mole) in a four-necked round bottomed flask equipped with a stirrer, a condenser, a dropping funnel and a thermometer. The addition of the Br₂ took place over one hour at 25°C, without any noticeable liberation of HBr from the reaction apparatus.
The progress of the reaction and its completion were followed by means of GC. Full conversion was achieved after two hours, after which the phases were separated. Crude 4,4'-dibromodiphenylether was obtained (324.8g) in a yield of 99% and a selectivity of 99.5%.

### Example 4

Example 2 was repeated, but using once CH₂Cl₂ and once CH₂Br₂ as the solvent, instead of EDC. Similar results were obtained.

### Example 5

Example 3 was repeated, but using once CH₂Cl₂ and once CH₂Br₂ as the solvent, instead of EDC. Similar results were obtained.

## Claims

1. A process for the preparation of 4,4'-dibromodiphenylether, wherein diphenylether is reacted with bromine and/or hydrobromic acid in the presence of one or more oxidizing agent(s), the reaction being carried out in a solvent comprising water and a water-immiscible organic solvent.

2. A process according to claim 1, wherein the reaction is carried out at a temperature in the range between -10° and 100°C.

3. A process according to claim 2, wherein the reaction temperature is in the range between 0° and 70°C.

4. A process according to any one of claims 1 to 3, wherein the oxidizing agent is selected from H₂O₂, NaBrO₃ and their mixtures.

5. A process according to claim 4, wherein the water-immiscible organic solvent is selected from the aliphatic or aromatic hydrocarbons and their mixtures, the said solvent being substantially unreactive under the bromination conditions.

6. A process according to any one of the previous claims, wherein the aqueous phase obtained after completion of the reaction is recycled to the next reaction batch, after a bleed of water has been effected, to remove excess water and accumulating salts, if formed in the reaction.

## Patentansprüche

1. Verfahren zur Herstellung von 4,4'-Dibromidiphenylether, worin Diphenylether mit Brom und/oder Bromwasserstoffsäure in Gegenwart von einem oder mehreren Oxidationsmittel(n) umgesetzt wird, wobei die Reaktion in einem Lösungsmittel durchegeführt wird, welches Wasser und ein nicht mit Wasser mischbares organisches Lösungsmittel umfaßt.

2. Verfahren nach Anspruch 1, woring die Reaktion bei einer Temperatur im Bereich zwischen -10°C und 100 °C durchgeführt wird.

3. Verfahren nach Anspruch 2, worin die Reaktionstemperatur im Bereich zwischen 0°C und 70°C liegt.

4. Verfahren nach einem der Ansprüche 1 bis 3, worin das Oxidationsmittel ausgewählt wird aus H₂O₂, NaBrO₃ und ihren Gemischen.

5. Verfahren nach Anspruch 4, worin das nicht Wasser mischbare organische Lösungsmittel ausgewählt wird aus den aliphatischen oder aromatischen Kohlenwasserstoffen und ihren Gemischen, wobei das Lösungsmittel unter den Bromierungsbedingungen im wesentlichent reaktionsufähig ist.

6. Verfahren nach einem der vorhergehenden Ansprüche, worin die wäßrige Phase, die nach der Beendigung der Reaktion erhalten wird, in die nächste Reaktionscharge zurückgeführt wird, nachdem Wasser entnommen worden ist, um überschüssiges Wasser und angesammelte Salze zu entfernen, sofern sie bei der Reaktion gebildet wurden.

## Revendications

1. Procédé de préparation de 4,4'-dibromo-diphényl-éther, dans lequel l'on fait réagir le diphényl-éther avec du brome et/ou de l'acide bromhydrique en présence d'un ou plusieurs agent(s) oxydant(s), la réaction étant conduite dans un solvant comprenant de l'eau et un solvant organique non miscible à l'eau.

2. Procédé selon la revendication 1, dans lequel la réaction est conduite à une température située dans l'intervalle compris entre -10° et 100°C.

3. Procédé selon la revendication 2, dans lequel la température du milieu réactionnel est située dans l'intervalle compris entre 0° et 70°C.

4. Procédé selon l'une quelconque des revendications 1 à 3, dans lequel l'agent oxydant est choisi parmi H₂O₂, NaBrO₃ et leurs mélanges.

5. Procédé selon la revendication 4, dans lequel le solvant organique non miscible à l'eau est choisi parmi les hydrocarbures aliphatiques ou aromatiques et leurs mélanges, ledit solvant étant pratiquement non réactif dans les conditions de bromuration.

6. Procédé selon l'une quelconque des revendications précédentes, dans lequel la phase aqueuse obtenue une fois la réaction terminée est réutilisée lors du prochain cycle réactionnel, après avoir pratiqué une purge d'eau, afin d'éliminer l'excès d'eau et les sels accumulés le cas échéant au cours de la réaction.
